# FASCICULE DE BREVET EUROPEEN

(11) **EP 0 324 669 B1**
(45) Date de publication et mention de la délivrance du brevet: **12.05.1993**
(21) Numéro de dépôt: 89400040.5
(22) Date de dépôt: 06.01.1989
(51) Int. Cl.: A61M 1/12

(54) **Prothèse cardiaque totalement implantable à membranes flottantes, à raccord rapide et à éléments sensibles amovibles**
Vollständig implantierbare Herzprothese mit schwimmenden Membranen, mit Schnellanschlüssen und mit auswechselbaren Elementen
Totally implantable cardiac prosthesis having floating membranes, quick connectors and replaceable elements

(30) Priorité: 14.01.1988 FR 8800381
(43) Date de publication de la demande: 19.07.1989
(73) Titulaire: UNIVERSITE PIERRE ET MARIE CURIE PARIS VI, 75230 Paris Cedex 05 (FR)
(72) Inventeur: Carpentier, Alain, F-75014 Paris (FR)
(74) Mandataire: Portal, Gérard

(56) Documents cités:
- EP-A- 0 228 950
- FR-A- 2 370 184
- FR-A- 2 585 250
- US-A- 3 030 892
- US-A- 3 478 695
- US-A- 4 397 049
- TRANSACTIONS AMERICAN SOCIETY FOR ARTIFICIAL INTERNAL ORGANS, vol. 31, 1-3 mai1985, pages 211-215, Atlanta, Georgia, US; T. TANAKA et al.: "Factors affecting left-right heart output differences in artificial heart implanted animals"
- TRANSACTIONS/AMERICAN SOCIETY FOR ARTIFICIAL INTERNAL ORGANS, vol. 27, 1981,pages 110-115, Washington, DC, US; R.L. WHALEN et al.: "Volume compensation forpulsatile blood pumps"

## Description

La présente invention concerne une prothèse cardiaque totalement implantable à membranes flottantes, à raccord rapide et à éléments sensibles amovibles.

Cette prothèse est destinée à une implantation soit temporaire en attente de transplantation cardiaque, soit définitive chez les malades ne pouvant bénéficier d'une transplantation pour des raisons médicales ou de tout autre nature.

De telles prothèses ont fait l'objet de plusieurs inventions antérieures par exemple celle décrite dans la demande de brevet FR 2 446 631.

Cette prothèse est essentiellement constituée par un ensemble monobloc biventriculaire qui comprend une coque étanche implantable dans la cavité péricardique, réalisée en un matériau compatible et non toxique vis-à-vis des tissus environnants et présentant une géométrie spécifique, qui reproduit la configuration du coeur naturel en deux chambres ventriculaires droite et gauche.

Cette prothèse contient : 1) un dispositif de pulsion du sang essentiellement constitué par deux membranes, dont l'une délimitant le ventricule droit travaille à l'élongation et dont la seconde délimitant le ventricule gauche travaille à la déformation; 2) des valves montées dans les orifices d'admission et de sortie ; 3) des moyens d'activation des membranes de pulsion précitées qui leur fournissent des pressions d'alimentation sensiblement équivalentes aux valeurs physiologiques ; 4) des moyens de régulation du débit cardiaque en fonction d'une part de la pression de remplissage et, d'autre part, de la pression aortique.

Cependant avec une telle prothèse a) le moyen d'activation et de régulation commandant les membranes de pulsion n'est pas monté directement sur la prothèse, ce qui augmente le volume de l'ensemble et complique son implantation, b) le moyen d'activation est unique pour les deux membranes rendant plus délicats et aléatoires les problèmes de régulation, c) la prothèse n'est pas biologisée et d) elle ne permet pas le remplacement de certains de ses éléments fonctionnels, opération qui peut s'avérer nécessaire dans le cas d'une panne ou d'une usure et qu'il est souhaitable de pouvoir faire sans changer toute la prothèse.

Le brevet FR-A-2 370 184 (NIKKISO) décrit une pompe pulsatoire pour circulation sanguine comportant deux membranes qui séparent une "chambre de transmission de pression". Ces deux membranes ont des mouvements différents, l'un de translation caractérisant le "diaphragme flexible", l'autre de ballonisation caractéristique de la forme en "calotte ou coupole". Leur fonction est de transformer une force mécanique en force hydraulique. Mais, l'appareil décrit n'est pas implantable, et n'est destiné qu'à prendre en charge la circulation sanguine de façon temporaire.

Le FR-A-2 585 250 décrit un coeur artificiel implantable comportant des orifices séparés de raccordement aux vaisseaux sanguins naturels mais ces orifices ne sont pas réunis sur un même connecteur, ce qui pose des problèmes d'encombrement et entraîne des risques importants d'hémorragies dans les zones de raccordement.

La présente invention permet de résoudre ces problèmes pour la première fois et de manière satisfaisante en proposant une prothèse cardiaque implantable constituée d'un module monocoque à raccord rapide comportant deux chambres ventriculaires biologisées complètement indépendantes et activées séparément, chaque chambre ventriculaire étant pourvue de deux orifices munis de valves - l'un des orifices servant de voie d'éjection, l'autre de voie d'admission du sang - et d'un dispositif d'activation et de régulation séparé constitué d'un organe électromécanique et d'un couple de membranes, la première membrane étant une membrane mécanique actionnée soit par un fluide de transmission mis sous pression au moyen dudit organe électromécanique, soit par un piston animé par un moteur suivant la modalité dite "pusher plate", et la seconde membrane, au contact du sang, étant une membrane biologique, flottante, se déplaçant sous l'action de la première membrane pendant la systole et sous l'action de la pression du sang pendant la diastole, caractérisée en ce que les voies d'admission des deux chambres ventriculaires sont regroupées et solidarisées sur une même lunette venant se raccorder de façon amovible et rapide sur un réceptacle suturé avec les oreillettes naturelles du patient.

Le déplacement des membranes nécessite d'une part un réservoir de fluide constitué par un sac déformable enveloppant la prothèse, d'autre part une chambre de compliance reliée à l'espace séparant les deux membranes. Cette dernière chambre par sa position sous-cutanée et donc facilement accessible par ponction transcutanée permet d'obtenir des renseignements utiles sur les pressions, les volumes et les modifications éventuelles du fluide intermembranaire. Elle permet aussi éventuellement sa communication permanente ou temporaire avec l'air libre.

La lunette cousue aux oreillettes et qui sert de réceptacle à la prothèse est d'une seule pièce et munie de dispositifs de suture assurant l'étanchéité. Elle peut être obturée temporairement par une plaque occlusive qui permet de vérifier avant l'implantation de la prothèse l'étanchéité du réceptacle.

La prothèse de l'invention comporte en outre sur la paroi des chambres ventriculaires ainsi que sur chacune des membranes, des capteurs destinés à déterminer à tout instant la position de la membrane flottante pour ajuster le débit et la fréquence de travail de l'organe électromécanique en vue de régler la course des membranes et la fréquence des battements.

Les membranes, les organes électromécaniques et les valves, éléments les plus sensibles à l'usure, sont montés sur le module de façon amovible pour permettre leur remplacement standard rapide.

L'originalité de la prothèse cardiaque de l'invention réside également dans la séparation complète des problèmes physiologiques et des problèmes mécaniques. La partie en contact avec le sang est constituée de matériaux et de composants hémocompatibles . Les organes électromécaniques ne sont pas en contact avec le sang, ce qui limite l'étendue des problèmes à résoudre. Un autre élément d'originalité est la conception monocoque avec une motorisation séparée des deux ventricules de façon à faciliter leur régulation respective.

Le module cardiaque proprement dit comporte donc deux chambres ventriculaires indépendantes ayant les fonctions de ventricule droit et de ventricule gauche, quatre orifices (une voie d'admission et une voie d'éjection pour chaque chambre), quatre valves (une pour chaque orifice) et deux membranes dans chaque chambre.

Les organes électromécaniques assurent, pour chaque chambre du module, l'actionnement des membranes à une fréquence donnée avec une course donnée, permettant ainsi la régulation des débits sanguins par variation de la fréquence et/ou des volumes déplacés.

Les organes électromécaniques de pulsion et électroniques de régulation sont totalement isolés du milieu sanguin. Ils sont miniaturisés, montés sur le module dans une disposition appendiculaire et dans les espaces libres entre les deux chambres ventriculaires. La disposition appendiculaire qui constitue un élément d'originalité de cette prothèse permet leur disposition transpéricardique dans les cavités pleurales, ce qui diminue considérablement l'encombrement intrapéricardique et facilite les échanges de chaleur entre les organes moteurs et les poumons.

Ce sont donc les membranes qui constituent l'interface physique" et biologique entre le milieu sanguin et les organes électromécaniques.

Le débit sanguin doit pouvoir être modifié en permanence en fonction de la demande de l'organisme soit par variation du volume d'admission et/ou par variation du volume d'éjection et/ou par variation de la fréquence.

Dans la prothèse, selon l'invention, le système de régulation de débit est le suivant : la membrane "mécanique" se déplace sous l'effet de l'actionneur électromécanique ou électro-hydraulique entre une position d'avancée (systole) et de retrait (diastole). Un premier élément de régulation est constitué par la possibilité pour cette membrane de parcourir la totalité ou seulement partie de sa course aussi bien pendant la systole que pendant la diastole. La membrane flottante ajoute pour sa part un deuxième élément de régulation complémentaire et plus sensible que le précédent. Son déplacement diastolique est fonction de la pression et du volume sanguin de remplissage. Un troisième élément de régulation est la fréquence de déplacement des membranes. Ces trois éléments de régulation sont mis en jeu soit passivement soit activement en fonction de renseignements obtenus par différents capteurs dont celui qui permet à tout moment de connaître la position de la membrane flottante n'est qu'un élément.

Les capteurs sont des capteurs de positionnement des deux membranes, des capteurs de pression et des capteurs de pression partielle d'oxygène situés dans les différentes cavités ou vaisseaux afférents ou efférents.

Le coeur est un générateur de débit pulsé et les courbes de pression sont entièrement déterminées par la force de pulsion sur les membranes, l'ouverture/fermeture des valves, les conditions hémodynamiques des réseaux d'amont et d'aval (pressions de compliance). La forme de la courbe pression de la prothèse selon l'invention reproduit donc au mieux la courbe d'évolution du volume refoulé en fonction du temps.

La membrane flottante dite membrane biologique qui constitue l'interface avec le sang est donc actionnée par une seconde membrane dite membrane mécanique. Cette dernière est elle-même actionnée par un piston du type dit "pusher plate" ou de préférence par un fluide qui permet une meilleure répartition des contraintes mécaniques. Les pressions générées dans les chambres ventriculaires sont de l'ordre en général de 100 à 140 mmHg pour le ventricule gauche et de 35 à 40 mmHg pour le ventricule droit. Le fluide dit de transmission est mû par l'organe électromécanique et plus particulièrement par une micropompe hydraulique volumétrique elle-même combinée à un micromoteur à courant continu sans balai ou moteur autosynchrone d'une puissance suffisante.

La variation de débit peut être réalisée par variation de la vitesse du moteur, la fréquence d'inversion de sens du moteur, ou par le changement de cylindrée de la pompe.

Ce micromoteur est immergé dans le liquide de transmission et possède une électronique de commande. Le liquide est stocké dans un réservoir constitué d'une enveloppe étanche déformable et non élastique entourant le module cardiaque et les organes électromécaniques et électroniques.

La solution hydraulique supprime les problèmes d'usure mécanique des membranes. La membrane mécanique n'est pas flottante et sa position dépend du volume refoulé par la pompe.

Le module cardiaque possède également des capteurs de pression sanguine, et d'évaluation de remplissage de chacun des ventricules ou du réservoir contenant le liquide de transmission. Le module cardiaque possède également des capteurs de mesure de la pression partielle d'oxygène des cavités gauches et droites utilisant des procédés colorimétriques.

La commande électronique du moteur autosynchrone exploite l'information de position donnée par les capteurs du rotor, réalise la synchronisation du champ tournant avec l'aimant permanent et amplifie le signal délivré aux bobines du stator.

Le moteur est régulé avec une carte électronique numérique possédant des circuits intégrés logiques qui permettent de reproduire le cycle cardiaque en modulant les durées d'inversion de sens et en contrôlant à chaque instant les vitesses de rotation, l'accélération et le freinage.

Cette carte est utilisée avec une carte électronique d'exploitation des signaux provenant des capteurs d'état cardiaque et avec l'électronique à microprocesseur qui réalise le contrôle et la régulation du débit cardiaque.

Les moteurs électriques, les pompes, les éléments électroniques et les capteurs sont immergés dans le liquide de transmission pour faciliter les charges thermiques et diminuer le bruit.

L'invention sera mieux comprise à la lecture de la description qui va suivre accompagnée des dessins annexés sur lesquels :

La figure 1 représente une vue d'ensemble.

La figure 2 est une vue en coupe transversale de la prothèse de l'invention représentant les positions systolique et diastolique de la membrane flottante.

La figure 3 est une vue de dessus du réceptacle sur lequel vient se raccorder de façon amovible la lunette d'admission du module.

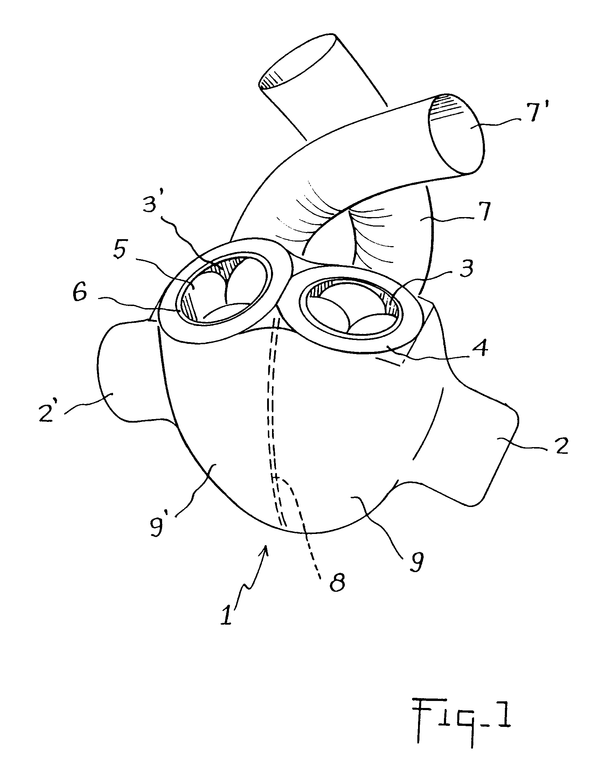

La figure 1 est une vue d'ensemble de la prothèse cardiaque selon l'invention.

Cette figure fait apparaître la disposition appendiculaire des organes électromécaniques 2,2' sur le module 1.

Les deux voies d'admission 3,3' regroupées côte à côte sur une seule lunette 4 sont munies de valves d'admission 5 et d'un élément d'étanchéité 6. La lunette 4 est destinée à venir se raccorder rapidement et facilement sur un réceptacle 30 (non représenté sur cette figure) suturé avec les oreillettes naturelles du patient. Les deux voies d'éjection 7,7' sont indépendantes et sont munies de valves d'éjection 5' (non représentées sur cette figure). Ces voies sont suturées directement avec les artères. La paroi 8 isole complètement le ventricule 9.

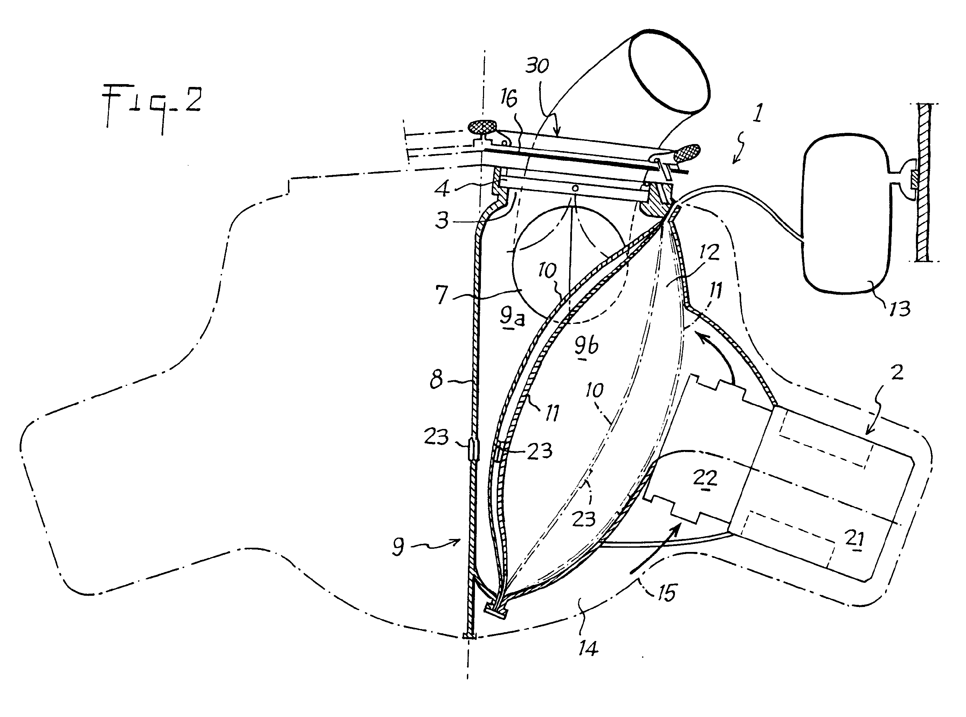

La figure 2 est une demi-coupe transversale de la prothèse de l'invention. Sur cette figure, seul le coeur gauche constitué du ventricule gauche 9 est représenté, la paroi 8 l'isolant complètement du ventricule droit 9'.

Le ventricule 9 est divisé en deux parties : une chambre biologique 9a et une chambre mécanique 9b, l'interface entre ces deux chambres étant constituée par une membrane biocompatible mobile 10. C'est donc la position de cette membrane 10 qui détermine le volume de la chambre biologique 9a et par suite la quantité de sang admise ou éjectée. La membrane 10 est flottante, c'est-à-dire mobile unilatéralement entre la position systolique correspondant à la fin de la période d'éjection, moment où la chambre biologique 9a a un volume minimum, et la position diastolique correspondant à la fin de la période d'admission, moment où la chambre biologique 9a a un volume maximum (de l'ordre de 60 à 80 cc), selon la taille de la prothèse.

La membrane 10 est actionnée par une seconde membrane dite membrane mécanique 11. Pendant la phase d'éjection, la membrane 11 repousse la membrane 10 depuis la position diastolique jusqu'à la position systolique puis pendant la phase d'admission la membrane 10 revient à la position diastolique sous le simple effet de la pression sanguine.

La position diastolique de la membrane 10 correspond à un équilibre de part et d'autre de ladite membrane entre la pression sanguine de remplissage (qui est de l'ordre de 10 mmHg pour le coeur gauche et de 8 mmHg pour le coeur droit) et la chambre de compliance.

Il existe entre la membrane biologique 10 et la membrane mécanique 11 un volume libre intermédiaire 12 occupé par un fluide maintenu lors de la diastole à une pression légèrement inférieure ou égale à la pression de remplissage du coeur droit ou du coeur gauche. Ce volume libre 12 est en communication avec une chambre de compliance 13 facilement accessible de l'extérieur du corps afin de pouvoir le cas échéant, réajuster la pression de volume libre intermédiaire.

La membrane mécanique 11 est elle-même actionnée au moyen d'un organe électromécanique 2 par l'intermédiaire d'un fluide de transmission 14 (et de préférence un liquide) stocké dans le réservoir constitué par une enveloppe déformable étanche 15 entourant à la fois le module 1, les organes électromécaniques 2,2' et les éléments de régulation. Cette disposition permet de réduire le bruit des éléments mécaniques et favorise les échanges thermiques.

L'organe électromécanique 2 est donc immergé et il se compose d'un micromoteur électrique 21 combiné dans le cas de figure à une micropompe hydraulique 22. Le micromoteur 21 peut être par exemple un moteur autosynchrone. Pendant la phase d'éjection, la micropompe 22 aspire le liquide 14 directement dans le réservoir 15 et le refoule en un temps très court contre la membrane mécanique 11 pour la repousser au contact de la membrane biologique 10. Pendant la phase d'admission, la micropompe 22 aspire le liquide 14 précédemment au contact de la membrane 11 pour la refouler dans le réservoir 15, ce qui a pour effet de faire revenir la membrane 11 à sa position de repos. La fréquence des battements cardiaques correspond donc à la fréquence d'inversion du sens de rotation de la micropompe 22. La course de la membrane 10 dépend de la course de la membrane 11 et donc du volume de liquide 14 refoulé par la micropompe 22 mais aussi de la pression de remplissage ventriculaire. Le micromoteur 21 et la micropompe 22 sont commandés par un dispositif électronique de contrôle et de régulation comprenant un jeu de capteurs analogiques 23 montés notamment sur la paroi 8 et sur les membranes 10, 11,afin de connaître de façon continue la position des membranes, la fréquence des battements, la pression sanguine à l'intérieur de la chambre ventriculaire et de la chambre de compliance, la pression du liquide de transmission, la pression partielle d'oxygénation, etc. Les informations fournies par les capteurs 23 sont utilisées par le dispositif de contrôle électronique pour modifier ou ajuster de façon automatique les paramètres de fonctionnement du module 1 à des valeurs prédéterminées.

Le ventricule 9, en référence aux figures 1 et 2, possède deux orifices, l'un servant de voie d'admission 3 et l'autre de voie d'éjection 7. Ces orifices sont respectivement munis d'une valve d'admission 5 et d'une valve d'éjection 5' et font communiquer le ventricule 9 avec le reste du système circulatoire. La voie d'éjection 7 est destinée à être suturée directement avec une artère.

Les voies d'admission 3,3' des ventricules 9,9' sont regroupées et solidarisées sur une même lunette 4.

La lunette 4 vient elle-même se raccorder de façon amovible et rapide sur le réceptacle 30 suturé avec les oreillettes naturelles du patient.

Tous les éléments fragiles ou susceptibles d'être usés rapidement, tels que les valves, micromoteurs, micropompes et membranes sont montés sur le module de façon amovible afin de pouvoir être remplacés facilement sans changer tout le module 1.

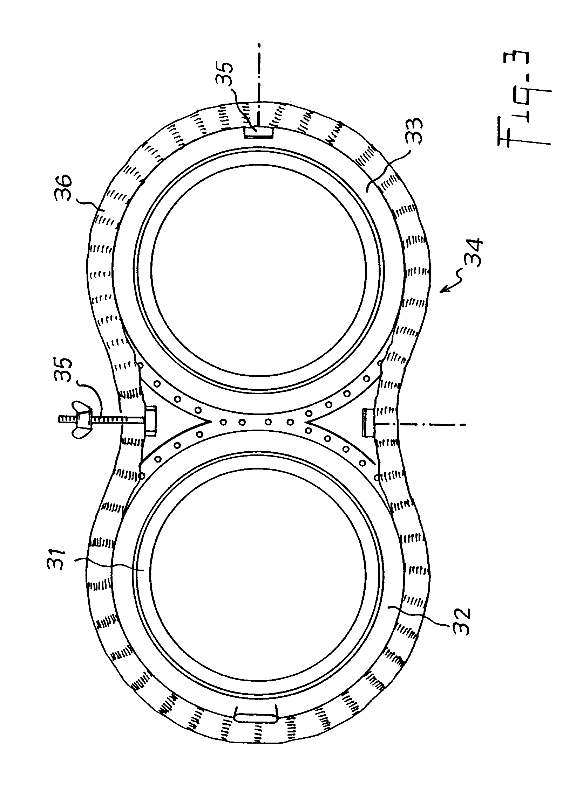

Le réceptacle 30 est constitué d'une lunette symétrique 34 de la lunette 4 et sur laquelle vient se raccorder le module 1 de façon amovible et rapide. Son originalité tient à ce qu'il est d'une seule pièce réunissant les deux orifices d'admission 32,33. Il comporte un élément de suture constitué par exemple d'un anneau périphérique de suture 36 en tissu (par exemple du Dacron), complété par une bande de fixation interorificielle de même tissu. La lunette 34 comporte comme illustré sur la figure 3 des perforations régulièrement espacées pour le passage des fils de suture et une encoche destinée à loger les noeuds des fils de suture pour éviter de gêner la jonction parfaite entre le réceptacle et la prothèse. Ce réceptacle peut être occlus par une plaque 16 (voir figure 2) pour vérifier l'étanchéité des sutures. Il possède des joints d'étanchéité avec la prothèse et des éléments de fixation rapide 35.

## Revendications

1. Prothèse cardiaque implantable constituée d'un module monocoque (1) à raccord rapide comportant deux chambres ventriculaires biologisées, indépendantes (9,9') activées séparément, chacune étant pourvue de deux orifices munis de valves (5,5'), l'un des orifices servant de voie d'éjection (7,7') et l'autre de voie d'admission (3,3') du sang, et d'un dispositif d'activation et de régulation séparé constitué d'un organe électromécanique (2), actionnant un couple de membranes (10,11), la première membrane étant une membrane mécanique (11) actionnée par un piston ou un fluide de transmission (14) mis sous pression au moyen dudit organe électromécanique (2) et la seconde membrane étant une membrane biologique flottante (10), au contact du sang se déplaçant sous l'action de la première membrane (11) entre les positions systolique et diastolique, caractérisée en ce que les voies d'admission (3,3') des deux chambres ventriculaires (9,9') sont regroupées et solidarisées sur une même lunette (4), venant se raccorder de façon amovible et rapide sur un réceptacle (30) suturé avec les oreillettes naturelles du patient.

2. Prothèse cardiaque selon la revendication 1, caractérisée en ce que le réceptacle (30) comporte deux orifices d'admission auriculaires qui sont regroupés et solidarisés sur une même lunette (34) qui comporte deux parties inclinées vers le bas de part et d'autre de l'axe de révolution de la prothèse ; chacune desdites parties étant associée à l'une des voies d'admission et ladite lunette (34) étant raccordée d'une part avec les oreillettes au moyen d'éléments de suture et d'autre part avec la prothèse au moyen d'une connexion rapide et réversible.

3. Prothèse cardiaque selon la revendication 2, caractérisée en ce que lesdits éléments de suture sont constitués d'un anneau périphérique (36) et d'une bande de fixation interorificielle et en ce que ladite lunette (34) comporte des perforations régulièrement espacées pour le passage des fils de suture et une encoche destinée à loger les noeuds des fils de suture pour éviter de gêner la jonction parfaite entre le réceptacle et la prothèse.

4. Prothèse selon l'une des revendications précédentes, caractérisée en ce qu'une plaque d'occlusion (16) des orifices auriculaires peut être fixée pour vérifier l'étanchéité des sutures avant de pratiquer le raccordement avec la prothèse.

5. Prothèse cardiaque selon l'une des revendications précédentes, caractérisée en ce que les deux membranes (10,11) définissent entre elles en position diastolique une enceinte (12) occupée par un fluide et communiquant avec une chambre de compliance (13), le volume de ladite enceinte (12) étant fonction de la pression de remplissage de la chambre ventriculaire.

6. Prothèse cardiaque selon l'une des revendications précédentes, caractérisée en ce que la chambre de compliance (13) est facilement accessible de l'extérieur du corps pour ajuster son remplissage.

7. Prothèse cardiaque selon la revendication 6 , caractérisée en ce que ladite chambre de compliance peut être mise en contact avec l'air libre.

8. Prothèse cardiaque selon l'une des revendications précédentes, caractérisée en ce qu'elle comporte en outre sur la paroi (8) des chambres ventriculaires (9,9') ainsi que sur chacune des membranes (10,11), des capteurs (23) destinés à déterminer à tout instant la position de la membrane flottante (10) pour ajuster le débit et la fréquence de travail de l'organe électromécanique (2) en vue de régler la course des membranes et la fréquence des battements.

9. Prothèse cardiaque selon l'une des revendications précédentes, caractérisée en ce que l'organe électromécanique (2) est constitué d'un micromoteur électrique (21) accouplé avec une micropompe (22) à débit réglable, volumétrique, à engrenage.

10. Prothèse cardiaque selon l'une des revendications pécédentes, caractérisée en ce que les membranes (10,11), les organes électromécaniques (2) et les valves (5,5') sont montés sur le module (1) de façon amovible pour permettre leur remplacement rapide.

11. Prothèse cardiaque selon l'une des revendications précédentes, caractérisée en ce que les membranes (10), les valves et le revêtement interne de la prothèse sont biologisés.

12. Prothèse cardiaque selon l'une des revendications précédentes, caractérisée en ce que les organes électromécaniques (2) sont montés latéralement sur le module (1) dans une disposition appendiculaire permettant leur positionnement en dehors du sac péricardique.

13. Prothèse cardiaque selon l'une des revendications précédentes, caractérisée en ce que le réservoir de liquide de transmission (14) est constitué par une enveloppe étanche déformable et non élastique (15) entourant latéralementle module (1), l'organe électromécanique (2) et les éléments électroniques.

14. Prothèse cardiaque selon l'une des revendications précédentes, caractérisée en ce que les moteurs électriques, les pompes, les éléments électroniques et les capteurs sont immergés dans le liquide de transmission pour faciliter les charges thermiques et diminuer le bruit.

15. Prothèse cardiaque selon l'une des revendications précédentes, caractérisée en ce que les capteurs sont des capteurs de positionnement des deux membranes, des capteurs de pression et des capteurs de pression partielle d'oxygène situés dans les différentes cavités ou vaisseaux afférents ou efférents.

## Patentansprüche

1. Implantierbare Herzprothese, bestehend aus einem Einschalenelement (1) mit Schnellverbindung, welches zwei unabhängige, separat betätigte biologisch kompatible Herzkammern (9,9') aufweist, die jeweils mit zwei mit Ventilen (5,5') ausgestatteten Öffnungen versehen sind, wobei eine der Öffnungen als Blutausströmbahn (7,7') und die andere als Bluteinströmbahn (3,3') dient, und mit einer separaten Betätigungs- und Regulierungseinrichtung, bestehend aus einem elektromechanischen Organ (2) zur Betätigung eines Paares Membranen (10,11), wobei die erste Membran eine mechanische Membran (11) ist, betätigt durch einen Kolben oder ein mittels des elektromechanischen Organs (2) unter Druck gesetztes Übertragungsfluid (14), und die zweite Membran eine mit dem Blut in Kontakt befindliche biologische Schwimmembran ist, die sich unter der Wirkung der ersten Membran (11) zwischen der Systolenposition und der Diastolenposition hin- und herbewegt, dadurch gekennzeichnet, daß die Bluteinströmbahnen (3,3') der beiden Herzkammern (9,9') auf ein- und derselben Ringhalterung (4) angeordnet und befestigt sind, welche abnehmbar und schnell an einen mit den natürlichen Herzvorhöfen des Patienten vernähten Auffang anschließbar ist.

2. Herzprothese nach Anspruch 1, dadurch gekennzeichnet, daß der Auffang (30) zwei Aurikeleinströmöffnungen aufweist, die auf ein- und derselben Ringhalterung (34) angeordnet und befestigt sind, welche zwei beiderseits der Drehachse der Prothese nach unten geneigte Teile aufweist; wobei jeder dieser Teile einer der Einströmbahnen zugeordnet ist und die Ringhalterung (34) einerseits durch Nahtelemente mit den Aurikeln und anderseits durch eine Schnell- und Umkehrverbindung mit der Prothese verbunden ist.

3. Herzprothese nach Anspruch 2, dadurch gekennzeichnet, daß die Nahtelemente durch einen peripheren Ring (36) und ein Fixierband zwischen den Öffnungen gebildet sind, und daß die Ringhalterung (34) zur Vermeidung einer Irritation der einwandfreien Verbindung zwischen Auffang und Prothese gleichmäßig im bestand vorgesehene Perforationen für den Durchtritt der Nahtfäden und eine Aussparung zur Aufnahme der Knoten der Nahtfäden aufweist.

4. Prothese nach einem der vorhergehenden Ansprüche, dadurch gekennzeichnet, daß eine Verschlußplatte (16) für die Aurikelöffnungen zur Überprüfung der Dichtheit der Nähte vor Herstellung der Verbindung mit der Prothese anbringbar ist.

5. Herzprothese nach einem der vorhergehenden Ansprüche, dadurch gekennzeichnet, daß die beiden Membranen (10,11) in der Diastolenposition dazwischen ein Behältnis (12) bilden, das mit einem Fluid gefüllt ist und mit einer Ausgleichskammer (13) kommuniziert, wobei das Volumen des Behältnisses (12) Funktion des Fülldrucks der Herzkammer ist.

6. Herzprothese nach einem der vorhergehenden Ansprüche, dadurch gekennzeichnet, daß die Ausgleichskammer (13) zur Einstellung ihres Füllstandes von außerhalb des Körpers leicht zugänglich ist.

7. Herzprothese nach Anspruch 6, dadurch gekennzeichnet, daß zwischen der Ausgleichskammer und dem Freien ein Kontakt herstellbar ist.

8. Herzprothese nach einem der vorhergehenden Ansprüche, dadurch gekennzeichnet, daß sie weiters an der Wand (8) der Herzkammern (9,9') sowie an jeder der Membranen (10,11) Sensoren (23) für die ständige Ermittlung der Position der Schwimmembran (10) zum Einstellen der Durchflußmenge und Arbeitsfrequenz des elektromechanischen Organs (2) zwecks Regulierens der Membranbewegungen und der Schlagfrequenz umfaßt.

9. Herzprothese nach einem der vorhergehenden Ansprüche, dadurch gekennzeichnet, daß das elektromechanische Organ (2) durch einen Elektromikromotor (21) gebildet ist, der an eine Mikroförderpumpe (22) mit regulierbarer Durchflußmenge mit Zahnrad gekoppelt ist.

10. Herzprothese nach einem der vorhergehenden Ansprüche, dadurch gekennzeichnet, daß die Membranen (10,11), die elektromechanischen Organe (2) und die Ventile (5,5') zur Ermöglichung ihres raschen Austausches abnehmbar am Element (1) montiert sind.

11. Herzprothese nach einem der vorhergehenden Ansprüche, dadurch gekennzeichnet, daß die Membranen (10), die Ventile und die Innenverkleidung der Prothese biologisch kompatibel sind.

12. Herzprothese nach einem der vorhergehenden Ansprüche, dadurch gekennzeichnet, daß die elektromechanischen Organe (2) in einer appendixartigen Anordnung seitlich am Element (1) montiert sind, wodurch ihre Positionierung außerhalb des Herzbeutels möglich ist.

13. Herzprothese nach einem der vorhergehenden Ansprüche, dadurch gekennzeichnet, daß das Reservoir für die Übertragungsflüssigkeit (14) durch eine verformbare und nicht elastische dichte Hülle (15) gebildet ist, die das Element (1), das elektromechanische Organ (2) und die elektronischen Elemente seitlich umgibt.

14. Herzprothese nach einem der vorhergehenden Ansprüche, dadurch gekennzeichnet, daß die Elektromotoren, die Pumpen, die elektronischen Elemente und die Sensoren zur Erleichterung der Wärmebelastungen und Verringerung der Geräusche in der Übertragungsflüssigkeit versenkt sind.

15. Herzprothese nach einem der vorhergehenden Ansprüche, dadurch gekennzeichnet, daß die Sensoren Positionsaufnehmer der beiden Membranen, Druckaufnehmer und Sauerstoffteildruckaufnehmer sind, welche sich in den verschiedenen Hohlräumen oder zuführenden oder wegführenden Gefäßen befinden.

## Claims

1. Implantable cardiac prosthesis constituted by a quick-connect, one-piece module (1) comprising two ventricular chambers, rendered biological, independent (9,9') and activated separately, each ventricular chamber being provided with two orifices provided with valves (5,5'), one of the orifices serving as ejection duct (7,7') and the other as inlet duct (3,3') for the blood, and with a separate activation and regulation device constituted by an electro-mechanical member (2) actuating a pair of membranes (10,11), the first membrane being a mechanical membrane (11) actuated by a piston or a transmission fluid (14) pressurized by means of said electro-mechanical member (2), and the second membrane, in contact with the blood, being a floating biological membrane (10), moving under the action of the first membrane (11) between the diastole and systole positions, characterized in that the admission ducts (3,3') of the two ventrical chambers (9,9') are grouped and connected on the same bezel (4), said bezel being removably and quickly connected to a receptacle (30) sutured on the patient's natural atria.

2. Cardiac prosthesis according to claim 1, characterized in that the receptacle (30) comprises two auricular admission ducts which are grouped and connected on the same bezel (34), the latter comprising two parts which are inclined downward on either side of the axis of revolution of the prosthesis; each of said parts being associated to one of the inlet ducts and said bezel (34) being connected, on the one hand, to the atria by means of suture elements and, on the other hand, to the prosthesis by means of a quick and reversible connection.

3. Cardiac prosthesis according to claim 2, characterized in that said suture elements are constituted by a peripheral ring (36) and by an inter-orifice fastening tape and in that said bezel (34) comprises regularly spaced apart perforations for the passage of the suture threads and a notch adapted to house the knots of the suture threads to ensure perfect join between the receptacle and the prosthesis.

4. Prosthesis according to one of the preceding claims, characterized in that a plate (16) for occluding the auricular ducts may be fixed thereto in order to check tightness of the sutures before effecting connection with the prosthesis.

5. Cardiac prosthesis according to one of the preceding claims, characterized in that the two membranes (10,11) define therebetween in diastolic position an enclosure (12) occupied by a fluid and communicating with a compliance chamber (13), the volume of said enclosure (12) being a function of the filling pressure of the ventricular chamber.

6. Cardiac prosthesis according to one of the preceding claims, characterized in that the compliance chamber (13) is easily accessible from outside the body in order to adjust filling thereof.

7. Cardiac prosthesis according to claim 6, characterized in that said compliance chamber may be placed in contact with the free air.

8. Cardiac prosthesis according to one of the preceding claims, characterized in that it further comprises, on the wall (8) of the ventricular chambers (9,9') as well as on each of the membranes (10,11), sensors (23) adapted to determine at any instant the position of the floating membrane (10) in order to adjust the output and working frequency of the electro-mechanical member (2) with a view to regulating the stroke of the membranes and the frequency of the beats.

9. Cardiac prosthesis according to one of the preceding claims, characterized in that the electromechanical member (2) is constituted by an electric micromotor (21) coupled with a positive displacement micropump (22) with adjustable output and with gear.

10. Cardiac prosthesis according to one of the preceding claims, characterized in that the membranes (10,11), the electro-mechanical members (2) and the valves (5,5') are removably mounted on the module (1) in order to allow rapid filling thereof.

11. Cardiac prosthesis according to one of the preceding claims, characterized in that the membranes (10), the valves and the inner coating of the prosthesis are rendered biological.

12. Cardiac prosthesis according to one of the preceding claims, characterized in that the electromechanical members (2) are mounted laterally on the module (1) in an appendicular arrangement allowing their positioning outside the pericardiac sac.

13. Cardiac prosthesis according to one of the preceding claims, characterized in that the reservoir of transmission liquid (14) is constituted by a deformable, non-elastic tight envelope (15) laterally surrounding the module (1), the electro-mechanical member (2) and the electronic elements.

14. Cardiac prosthesis according to one of the preceding claims, characterized in that the electric motors, the pumps, the electronic elements and the sensors are immersed in the transmission liquid in order to facilitate heat loads and to reduce noise.

15. Cardiac prosthesis according to one of the preceding claims, characterized in that the sensors are sensors for positioning the two membranes, sensors of partial oxygen pressure, located in the different afferent or efferent cavities or vessels.
